(19) **Europäisches Patentamt / European Patent Office / Office européen des brevets**

(11) **EP 1 799 193 B1**

(12) **FASCICULE DE BREVET EUROPEEN**

(45) Date de publication et mention
de la délivrance du brevet:
**14.05.2014  Bulletin 2014/20**

(21) Numéro de dépôt: **05807821.3**

(22) Date de dépôt: **05.10.2005**

(51) Int Cl.:
*A61K 9/14* (2006.01)     *A61K 9/50* (2006.01)
*A23K 1/175* (2006.01)     *A23L 1/00* (2006.01)

(86) Numéro de dépôt international:
**PCT/FR2005/002441**

(87) Numéro de publication internationale:
**WO 2006/037891 (13.04.2006 Gazette 2006/15)**

(54) **ENCAPSULATION D ' EXTRAITS VEGETAUX HYDROPHOBES ADSORBES ET/OU ABSORBES DANS DE LA SILICE PRECIPITEE**

EINKAPSELUNG VON ADSORBIERTEN ODER IN FÄLLUNGSKIESELSÄURE ABSORBIERTEN HYDROPHOBISCHEN PFLANZENEXTRAKTEN

ENCAPSULATING HYDROPHOBIC PLANT EXTRACTS ADSORBED AND/OR ABSORBED IN PRECIPITATED SILICA

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **05.10.2004  FR 0410618**

(43) Date de publication de la demande:
**27.06.2007  Bulletin 2007/26**

(73) Titulaire: **PANCOSMA FRANCE S.A.S.
01200 Bellegarde sur Valserine (FR)**

(72) Inventeurs:
- **LOMBARDY ALRIC, Monique**
  **F-63000 Clermont-Ferrand (FR)**
- **CARDOT, Jean-Michel**
  **F-63122 Ceyrat (FR)**
- **GAUTIER, François**
  **F-74600 Seynod (FR)**
- **KAMEL, Christopher**
  **ES-43892 Miami Playa (ES)**

- **MAZURANOK, Laurence**
  **F-01350 Culoz (FR)**
- **MEUNIER, Jean-Philippe**
  **F-01170 Cessy (FR)**

(74) Mandataire: **Poncet, Jean-François
Cabinet Poncet
7, chemin de Tillier
B.P. 317
74008 Annecy Cedex (FR)**

(56) Documents cités:
| | |
|---|---|
| **EP-A- 0 345 109** | **EP-A- 1 132 009** |
| **EP-A- 1 297 751** | **WO-A-99/07237** |
| **WO-A-03/009710** | **WO-A-2004/016288** |
| **WO-A-2004/073689** | **DE-A1- 4 218 768** |
| **DE-A1- 10 211 195** | **GB-A- 1 125 882** |
| **US-A- 3 413 118** | **US-A- 4 519 961** |
| **US-A- 4 797 288** | **US-A- 4 842 863** |
| **US-A- 5 227 166** | **US-A1- 2003 003 040** |
| **US-A1- 2003 215 547** | |

## Description

DOMAINE TECHNIQUE DE L'INVENTION

La présente invention concerne les extraits végétaux utilisés comme additifs alimentaires dans la nutrition animale ou la médecine vétérinaire, pour améliorer les performances zootechniques et/ou la santé des animaux.

On utilise généralement soit des extraits végétaux naturels, soit des produits "naturels identiques". Les produits "naturels identiques" sont obtenus par synthèse, et présentent une structure moléculaire identique à celle d'extraits naturels.

Dans la description de la présente demande de brevet, et dans les revendications, l'expression "extraits végétaux" désignera indifféremment les extraits végétaux naturels et les produits naturels identiques, et l'expression "liquide hydrophobe" désignera un liquide insoluble ou peu soluble dans l'eau, c'est-à-dire dont la solubilité est inférieure à dix grammes par litre d'eau.

Un grand nombre de ces extraits végétaux proviennent de la séparation d'un ou plusieurs constituants liquides ou solides issus d'une matière première par entraînement à la vapeur ou dissolution dans un fluide, sont des huiles essentielles, des résines, des oléorésines, des arômes, et existent sous forme liquide, voire pâteuse. Le caractère souvent lipophile de ces constituants les rend également insolubles ou peu solubles dans l'eau.

Ces extraits végétaux sont utilisés en faible quantité, et doivent être mélangés à un substrat alimentaire, pour l'administration animale.

Lorsque l'extrait végétal est sous forme liquide, il est souvent difficile à disperser de façon homogène dans le substrat alimentaire. C'est la raison pour laquelle on préfère un extrait végétal sous forme de poudre, dans laquelle l'extrait végétal est fixé sur un substrat. Mais sa conservation ne peut être assurée que pendant une période de temps relativement courte, car un stockage prolongé de quelques mois, voire de quelques semaines, entraîne une perte de ses propriétés qui sont souvent liées à des composants très volatiles, hygroscopiques ou sensibles à l'oxydation.

Dans les extraits végétaux utilisés, un certain nombre de molécules peuvent être toxiques ou irritantes pour le manipulateur, peuvent poser à l'aliment des problèmes de goût et odeur diminuant son appétence, peuvent être incompatibles avec un co-ingrédient utilisé dans les pré-mix ou dans l'aliment, et peuvent être sensibles aux différents processus de fabrication de l'aliment, par exemple lors d'une granulation à la vapeur ou lors de l'extrusion.

Pour un certain nombre d'applications, l'action d'extraits de plantes peut être optimisée de façon significative par un système de protection qui permet de cibler un site de libération déterminé.

Pour les raisons ci-dessus, on a développé depuis de nombreuses années diverses techniques d'encapsulation des extraits végétaux pulvérulents. On a ainsi proposé des techniques de séchage par pulvérisation (spray drying), de refroidissement par pulvérisation (spray cooling), d'extrusion, de granulation, d'adsorption et/ou absorption sur support (silice, sel, remoulages, rafles de maïs, maltodextrines) ou d'enrobage par lit d'air fluidisé.

Toutefois, l'état de l'art généralement admis met en évidence la difficulté d'utilisation industrielle de la technique de lit d'air fluidisé pour enrober un produit pulvérulent à base d'un ou plusieurs extraits végétaux pour la nutrition animale ou la médecine vétérinaire, notamment dans le cas d'extraits végétaux hydrophobes.

En particulier, l'enrobage par la technique du lit d'air fluidisé apparaît inapplicable pour protéger des extraits végétaux existant sous forme de liquide hydrophobe. En effet, dans ce cas, il est difficile de trouver un support adéquat afin de fixer le liquide sur un solide avant une étape d'enrobage, sans recourir à une étape préalable et onéreuse de granulation. Le taux d'absorption du liquide sur le solide reste généralement faible, inférieur à 30 % en poids, et le support généralement utilisé nécessite des épaisseurs d'enrobage trop importantes qui rendent le procédé inapplicable industriellement pour une utilisation en nutrition animale ou en médecine vétérinaire.

Ces difficultés ont conduit jusqu'à présent à préférer les techniques de séchage par pulvérisation, de refroidissement par pulvérisation, d'extrusion, de granulation ou d'adsorption et/ou absorption sur support. Cependant, le séchage par pulvérisation et le refroidissement par pulvérisation conduisent à la production de poudres trop fines, qui ne satisfont pas les conditions de sécurité d'utilisation. L'extrusion ne permet pas, non plus, d'obtenir une poudre présentant les propriétés physiques recherchées pour assurer un mélange homogène dans un substrat alimentaire.

Dans ces techniques, les extraits végétaux sous forme liquide sont incorporés dans une matrice afin de faciliter leur utilisation, mais la protection n'est pas totale. Il n'y a généralement pas de couche d'enrobage extérieur assurant la protection totale du principe actif vis-à-vis du milieu environnant. Il sera donc difficile d'envisager par exemple l'utilisation de ces techniques lorsque l'objectif est un contrôle du relargage du principe actif dans le tractus digestif.

Le document WO 2004/073689 A1 décrit et revendique des comprimés ou gélules obtenus par agglomération de particules de silice contenant un principe actif hydrophobe en solution dans une huile. Les particules sont de faible taille, comprise entre 2 $\mu$m et 400 $\mu$m, de préférence comprise entre 20 $\mu$m et 30 $\mu$m pour assurer une bonne agglomération. Les agglomérats peuvent être formés dans un lit fluidisé, puis éventuellement enrobés. Ces comprimés assurent une meilleure biodisponibilité du principe actif administré par voie orale.

Les documents US 2003/0003040 A1, WO 99/07237 A et EP 0 345 109 A1 décrivent des techniques de fabrication de silices précipitées ayant de bonnes capacités pour absorber des composés polaires.

Le document GB 1 125 882 A décrit l'utilisation d'un autre substrat, à savoir une pâte de silicate d'aluminium et de magnésium séchée et broyée, pour la réalisation de médicaments sous forme de granules ensuite enrobées.

Le document EP 1 132 009 A décrit l'utilisation d'un autre substrat, à savoir un silicate hydraté de magnésium ou d'aluminium, passé dans un mélangeur pour absorber un principe actif issu de plantes afin de réaliser un additif pour l'alimentation animale.

EXPOSE DE L'INVENTION

Le problème proposé par la présente invention est de concevoir une nouvelle structure de produits pulvérulents à base d'un ou plusieurs extraits végétaux pour la nutrition animale ou la médecine vétérinaire, permettant de :

- préserver l'intégrité du ou des principes actifs vis-à-vis de l'environnement pendant une période de stockage prolongée,
- protéger le ou les principes actifs lors de leur incorporation dans l'aliment,
- protéger l'environnement de la toxicité potentielle du ou des principes actifs,
- préserver le goût et l'odeur des aliments, en assurant leur appétence et en évitant de leur conférer un caractère irritant,
- assurer aux produits pulvérulents une bonne coulabilité, un poussiérage très faible voire nul, une densité élevée, une concentration élevée en principes actifs,
- contrôler la libération du ou des principes actifs dans le tractus digestif.

L'invention vise simultanément à améliorer l'efficacité des extraits végétaux sur l'animal, pour améliorer les performances zootechniques et/ou la santé des animaux.

Pour atteindre ces buts ainsi que d'autres, l'invention propose un produit pulvérulent à base d'un ou plusieurs extraits végétaux pour la nutrition animale ou la médecine vétérinaire, dans lequel :

- le ou les extraits végétaux sont sous forme de liquide hydrophobe,
- le ou les extraits végétaux sont adsorbés et/ou absorbés dans de la silice précipitée,
- la silice précipitée est sous forme de granules sphéroïdales non agglomérées,
- les granules sphéroïdales de silice précipitée ont une taille comprise entre 90 $\mu$m et 500 $\mu$m environ, de préférence comprise entre 200 $\mu$m et 500 $\mu$m environ, sensiblement dépourvues de fines particules c'est-à-dire de particules ayant une taille inférieure à 90 $\mu$m environ,
- les granules sphéroïdales de silice précipitée contenant le ou les extraits végétaux adsorbés et/ou absorbés sont enrobées d'une couche de matériau d'enrobage protecteur.
- le ou les extraits végétaux (1) sous forme liquide sont présents dans la silice précipitée (2) selon une proportion supérieure à 20 % en poids

La nature et la morphologie spécifique du substrat utilisé permettent de réaliser industriellement et à faible coût un enrobage protecteur efficace et non perturbateur, pour résoudre le problème ci-dessus.

De meilleurs résultats d'enrobage et de fluidité seront obtenus, dans le cas d'une production industrielle, en utilisant des granules sphéroïdales de silice précipitée de taille comprise entre 200 $\mu$m et 500 $\mu$m environ. On évite en particulier les risques d'agglomération lors de la réalisation de l'enrobage et les risques subséquents de défauts d'enrobage et de protection. En effet, des agglomérats de particules sphéroïdales présentent une forme aléatoire non sphéroïdale qui est moins adaptée à la réalisation d'un enrobage continu, et présentent une taille aléatoire. De tels agglomérats ne sont ainsi pas capables de résoudre le problème qui est à la base de l'invention.

De préférence, le ou les extraits végétaux sous forme liquide sont présents dans la silice précipitée selon une proportion supérieure à 20 % en poids, de préférence une proportion supérieure à 30 % en poids. On améliore ainsi la qualité et l'efficacité de l'enrobage, tout en optimisant l'efficacité du produit lui-même.

De bons résultats sont obtenus en prévoyant que les granulés sphéroïdales de silice précipitée ont une densité de remplissage à l'état tassé DRT supérieure à 0,29, une prise d'huile DOP supérieure à 100 millilitres/ 100 grammes, une surface BET comprise entre 140 et 240 m²/gramme environ, une surface spécifique CTAB comprise entre 140 et 230 m²/gramme environ, un taux d'humidité inférieur à 5 % en poids, un taux de refus au tamis d'au moins 92 % en poids pour des ouvertures de maille de 75 $\mu$m.

Pour réaliser une protection efficace, le matériau d'enrobage protecteur est de préférence présent en proportion d'environ 10 à 30 % en poids du produit pulvérulent.

On choisira le matériau d'enrobage protecteur en fonction des applications envisagées. Par exemple, le matériau d'enrobage protecteur peut assurer le masquage du goût ou des effets irritants des principes actifs contenus dans le ou les extraits végétaux. Selon un autre exemple, le matériau d'enrobage protecteur peut assurer la stabilité des principes actifs contenus dans le ou les extraits végétaux pendant un temps de stockage et/ou lors de procédés industriels d'utilisation du produit, notamment pour la fabrication d'aliments.

Selon une possibilité avantageuse, le matériau d'enrobage protecteur peut avoir la propriété de se dissoudre dans un milieu dont le pH est supérieur à un seuil de pH déterminé, de façon par exemple à être gastrorésistant. De façon plus générale, le matériau d'enrobage protecteur peut être choisi de façon à assurer une libération accélérée, retardée ou ciblée, dans le tractus digestif, des principes actifs contenus dans les extraits végétaux.

On peut par exemple utiliser un matériau d'enrobage protecteur choisi parmi les agents d'enrobage utilisés dans les domaines pharmaceutique ou agroalimentaire.

De bons résultats ont été obtenus en utilisant un matériau d'enrobage protecteur à base d'hydroxypropylmethylcellulose (HPMC) aqueuse.

Le matériau d'enrobage protecteur pourra par exemple être à base de gomme, ou de polysaccharides (amidon, cellulose), ou de protéine, ou de copolymères d'acide méthacrylique, ou de matière grasse, ou d'un mélange de ces produits.

Selon une possibilité avantageuse, le matériau d'enrobage protecteur peut avoir la propriété de contrôler le site de libération des principes actifs au cours de la digestion. De bons résultats ont été obtenus pour cela en utilisant un matériau d'enrobage protecteur à base d'une suspension d'éthylcellulose (EC).

En alternative, on peut utiliser un matériau d'enrobage protecteur à base de tout type de matériau d'enrobage classiquement utilisé tel que : graisse, gomme, amidon, protéine ... et leurs mélanges.

De bons résultats ont été obtenus en réalisant l'enrobage par une technique de lit d'air fluidisé. Dans ce cas, la couche de matériau d'enrobage protecteur présente les propriétés physico-chimiques d'une couche réalisée par lit d'air fluidisé : homogénéité de l'enrobage, continuité de l'enrobage.

Le ou les extraits végétaux peuvent être initialement sous forme de liquide hydrophobe tel que huile essentielle, oléorésine, gomme, résine, arôme.

L'invention propose également un procédé de fabrication d'un produit pulvérulent selon les caractéristiques ci-dessus, le procédé comprenant les étapes :

a) prévoir des granules sphéroïdales de silice précipitée de taille moyenne comprise entre 90 $\mu$m et 500 $\mu$m environ, de préférence comprise entre 200 $\mu$m et 500 $\mu$m environ, sensiblement dépourvues de fines particules,
b) prévoir un ou plusieurs extraits végétaux sous forme de liquide hydrophobe,
c) réaliser l'adsorption et/ou l'absorption d'une quantité appropriée du liquide hydrophobe dans les granules sphéroïdales de silice précipitée, en évitant leur agglomération,
d) appliquer, sur les granules sphéroïdales non agglomérées de silice précipitée contenant le liquide hydrophobe, au moins une couche de matériau d'enrobage protecteur par pulvérisation du matériau d'enrobage protecteur (3) liquide sur les granules sphéroïdales (2) dans un lit fluidisé (6).

L'étape c) d'adsorption et/ou d'absorption peut avantageusement être effectuée par pulvérisation dans un lit d'air fluidisé ou dans un mélangeur.

Lors de cette étape d'adsorption et/ou d'absorption, il est avantageux que le liquide hydrophobe soit adsorbé et/ou absorbé dans la silice précipitée selon une quantité d'au moins 20 % en poids, de préférence d'au moins 30 % en poids. De la sorte, on obtient une poudre de densité suffisante, qui est alors compatible avec la technique ultérieure d'enrobage par lit d'air fluidisé. Cela améliore la qualité et l'efficacité de l'enrobage.

Lors de l'étape d), l'enrobage des granules sphéroïdales peut avantageusement être réalisé par pulvérisation du matériau d'enrobage protecteur liquide sur les granules sphéroïdales dans un lit d'air fluidisé.

Lors de cette étape d'enrobage, le matériau d'enrobage protecteur liquide peut avantageusement être une solution aqueuse, ou une émulsion aqueuse, ou une graisse (lipide).

Pour que l'étape d'enrobage soit réalisée rapidement et à faible coût, il est avantageux que, dans le cas d'un matériau d'enrobage protecteur en solution aqueuse ou en émulsion aqueuse, la concentration en excipient d'enrobage soit de 8 % à 30 % en poids, et de préférence de 10 % à 25 % en poids.

On peut avantageusement prévoir, dans le matériau d'enrobage protecteur, la présence d'un plastifiant en proportion de 5 à 40 % en poids de matériau.

L'invention prévoit également l'application d'un tel produit à la nutrition animale, application dans laquelle on introduit en faible quantité le produit pulvérulent dans un substrat alimentaire. Si nécessaire, on mélange/disperse pour répartir équitablement le produit pulvérulent dans le substrat alimentaire. On améliore ainsi sensiblement l'efficacité de l'extrait végétal sur l'animal.

On peut toutefois appliquer l'invention en déposant l'additif sur l'aliment. Dans ce cas il n'y a pas de mélange homogène.

DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :

- la figure 1 est une vue en coupe diamétrale illustrant la structure d'un grain de poudre de produit selon un mode de réalisation de la présente invention ;
- la figure 2 illustre les étapes essentielles de fabrication du produit pulvérulent selon l'invention ;
- les figures 3 à 5 illustrent schématiquement trois modes de réalisation d'une technique de lit d'air fluidisé utilisable selon l'invention ; et
- la figure 6 est une vue schématique d'une installation de traitement par lit d'air fluidisé utilisable selon l'invention.

DESCRIPTION DES MODES DE REALISATION PREFERES

Depuis plusieurs années, on a mis en évidence de nombreuses propriétés d'extraits de plantes pour améliorer la santé ou les performances zootechniques des animaux. Par exemple, on a pu établir l'action d'extraits de plantes tels que des huiles essentielles sur les bactéries. Cette action peut être bactéricide et/ou bactériostatique. On peut citer par exemple l'action bactéricide du carvacrol, qui est le principal composant de l'huile essentielle d'origan.

A des concentrations faibles, ces extraits de plantes ont montré une action anti-oxydante ou stimulateur de l'activité de certaines populations bactériennes spécifiques. Ce phénomène a été démontré chez des animaux de laboratoire par la stimulation de production d'acide lactique par les lactobacilles en présence d'huile essentielle d'origan.

Le tableau ci-après donne une liste des effets de nombreux principes actifs extraits des plantes.

| Principes actifs | Plantes d'origines | propriétés |
|---|---|---|
| Phénols | | Anti-infectieux (large spectre : bactéricide, virucide, fongicide, parasiticide), immuno-stimulateur, tonique, stimulant général et du système nerveux central. |
| Carvacrol | Origan | + spasmolytique, anti-inflammatoire, anti-oxydant, anti-radicalaire, antiseptique, expectorant. |
| Thymol | Thym | + analgésique, sédatif, stimulateur digestif, anti-inflammatoire, anti-oxydant, anti-radicalaire, antiseptique. |
| Eugenol | Clou de girofle | + spasmolytique, carminatif, antiinflammatoire, anti-oxydant, anti-radicalaire, antiseptique, anti-spasmodique, anti-convulsivant, hépatoprotecteur, cholérétique. |
| Monoterpénols | | Anti-infectieux (large spectre), stimulant général et immunitaire. |
| Linalol | Bois de rose | + sédatif, spasmolytique |
| Menthol | Menthe | + hépatostimulant, vasoconstricteur, analgésique, stimulateur digestif, antiinflammatoire, carminatif, antiseptique, anti-spasmodique, expectorant, enterorelaxant, carminatif. |
| Aldéhyde Aromatique | | Anti-infectieux majeur, tonique général et du système nerveux sympathique |
| Cinnamaldéhyde | Cannelle | + anti-inflammatoire, anti-oxydant, anti-radicalaire, antiseptique spasmolytique, stimulateur digestif, cholérétique. |
| Oxydes | | Anti-infectieux, immuno-modulateur |
| Cinéole | Eucalyptus | + cholérétique, spasmolytique, antiseptique, stimulant du système nerveux central, sédatif. |
| Phénol méthyl-éther | | Analgésique, anti-inflammatoire, stimulateur digestif, antiseptique, spasmolytique, tonique, antalgique, tonifiant, anti-bactérien et anti-viral. |
| Trans-anéthole | Anis | + carminatif, stimulateur digestif |
| Estragole | Basilic | + stimulateur digestif |

(suite)

| Principes actifs | Plantes d'origines | propriétés |
|---|---|---|
| Ether-oxydes | | Antalgique, stimulateur digestif, antiseptique, spasmolytique, tonique |
| Apiole | Persil | + vasodilatoire |
| Composé sulfuré | | Stimulateur digestif, cholestérolytique, anti-inflammatoire |
| Allicine | Ail | |
| Alcaloïdes | | Action au niveau du système nerveux central, anesthésiant. |
| Sanguinarine | sanguinaire | Anti-microbien, anti-fongique, anti-inflammatoire. |
| Saponosides | | Molluscicide, anti-tussif, diurétique, anti-inflammatoire |
| Glycyrrhizine | réglisse | + Anti-diarrhérique, stimulateur digestif, anti-inflammatoire, anti-oxydant, immunostimulant. |

Ainsi, l'extrait végétal constitue le premier élément essentiel incorporé dans le produit selon l'invention.

Les extraits végétaux utilisés par l'invention sont le plus souvent sous forme de liquides hydrophobes. Il peut s'agir d'extraits naturels ou de produits de synthèse "naturels identiques".

Ils peuvent être sous forme d'huiles essentielles, de résines, d'oléorésines, d'arômes.

Le second élément essentiel rentrant dans la composition du produit selon l'invention est un substrat sous forme de granules sphéroïdales de taille appropriée.

La difficulté a été de trouver un substrat qui soit à la fois capable de contenir une quantité suffisante d'extraits végétaux, qui soit compatible avec la réalisation d'un enrobage protecteur efficace, et qui présente de bonnes propriétés de libération des principes actifs après administration à l'animal.

On utilise pour cela des granules sphéroïdales de silice précipitée.

Les extraits végétaux sont adsorbés et/ou absorbés dans les granules sphéroïdales de silice précipitée.

On a pu déterminer que la taille des granules sphéroïdales doit être comprise dans des limites déterminées pour obtenir les effets recherchés.

Ainsi, il est important que la taille des granules sphéroïdales soit inférieure à 500 $\mu$m environ, afin d'assurer une homogénéité satisfaisante du mélange ultérieur réalisé entre le produit pulvérulent et un substrat alimentaire pour l'animal.

Il est important d'autre part que la taille des granules sphéroïdales soit supérieure à 90 $\mu$m, pour éviter la présence des particules trop fines : de telles particules trop fines pourraient conduire d'une part à la présence de particules en suspension dans l'air lors de l'utilisation du produit, ce qui serait nocif pour les manipulateurs ; ces particules trop fines conduiraient d'autre part à perturber sensiblement le processus d'enrobage dans un lit d'air fluidisé, par la formation d'agglomérats. Une taille supérieure à 200 $\mu$m environ donne de meilleurs résultats, par une plus grande fiabilité de l'enrobage.

Il est ensuite avantageux de choisir des granules sphéroïdales de silice précipitée ayant une densité de remplissage à l'état tassé (DRT) supérieure à 0,29. La densité de remplissage à l'état tassé peut être déterminée selon la norme NF T 30-042.

Il est également avantageux de choisir une silice précipitée qui permette une prise d'huile hydrophobe DOP supérieure à 100 millilitres100 grammes. La prise de liquide hydrophobe DOP peut être mesurée selon la norme NF T 30-022 (mars 1953) en mettant en oeuvre le dioctylphtalate.

On peut également caractériser avantageusement les volumes poreux de la silice précipitée. Les volumes poreux sont mesurés par porosimétrie au mercure. Pour cela, la préparation de chaque échantillon peut se faire comme suit : chaque échantillon est préalablement séché pendant deux heures en étuve à 200°C, puis placé dans un récipient à essai dans les cinq minutes suivant sa sortie de l'étuve et dégazé sous vide, par exemple à l'aide d'une pompe à tiroirs rotatifs. Les diamètres de pores sont calculés par la relation de Washburn avec un angle de contact $\theta$ égal à 140° et une tension superficielle y égale à 484 dynes/cm. On peut utiliser un porosimètre de marque MICAOMERITICS 9300.

On peut également caractériser la silice précipitée par sa surface spécifique BET, que l'on choisira comprise entre 140 et 240 m$^2$/g environ. La surface spécifique BET est déterminée selon la méthode de BRUNAUER - EMMET - TELLER décrite dans -The journal of the American Chemical Society-vol. 60, page 309, février 1938, et correspondant à la norme NF T 45007 de novembre 1987.

On peut également caractériser la silice précipitée par sa surface spécifique CTAB, que l'on choisira comprise entre 140 et 230 m$^2$/g environ. La surface spécifique CTAB est la surface externe déterminée selon la norme 10 NF R 45007 de novembre 1987.

On cherchera également à s'assurer de l'absence, ou de la très faible proportion, de particules fines, en prévoyant des particules sphéroïdales dont le taux de refus est d'au moins 92 % en poids dans un tamis ayant une ouverture de maille de 75 μm.

Pour optimiser sa capacité d'adsorption et/ou d'absorption en extraits végétaux, la silice précipitée aura de préférence une humidité réduite : son taux d'humidité (perte au séchage à 105°C pendant 2 heures) sera de préférence inférieur à 5 % en poids, avant absorption des extraits végétaux.

On pourra également rechercher une silice précipitée ayant une friabilité moyenne inférieure à une limite admissible. La mesure de la friabilité de la silice précipitée est effectuée selon la méthode suivante : dans un appareil à lit d'air fluidisé, par exemple de marque Glatt GPCG1, on introduit une prise d'essai exactement pesée et égale à 800 grammes de silice précipitée, ayant une granulométrie définie, par exemple de 90 μm à 350 μm ; la silice est alors soumise à un lit d'air fluidisé avec une entrée d'air de 100 m³/heure, une température de 25°C et une pression d'air de 2,5 bars durant 30 minutes. Ensuite, l'échantillon est à nouveau tamisé sur un tamis de 90 μm, puis pesé. La friabilité F (pour cent) est donnée par l'expression :

$$F=(1-B/A)100\%$$

avec A = poids de la silice retenue par le tamis 90 μm avant essai
avec B = poids de la silice retenue par le tamis 90 μm après essai.

On pourra également caractériser la silice précipitée par sa coulabilité. La coulabilité est illustrée par le temps d'écoulement des compositions conditionnées, et est mesurée par passage de 50 grammes de produit à travers un silo en verre d'orifice calibré (diamètre du cylindre : 50 mm ; hauteur du cylindre : 64 mm ; angle de cône : 53° ; diamètre de passage à la base du cône : 8 mm). Selon cette méthode, on remplit le silo, fermé à sa base, à l'aide de 50 grammes de produit ; puis on ouvre la base et on note le temps d'écoulement complet des 50 grammes du produit.

On peut également apprécier la coulabilité par mesure de l'angle de talus selon la norme NF T 20-221.

Le troisième élément essentiel rentrant dans la composition du produit pulvérulent selon l'invention est l'enrobage protecteur. L'enrobage protecteur a pour fonction d'isoler les extraits végétaux par rapport au milieu ambiant, pendant toute la période préliminaire au cours de laquelle on veut éviter le contact avec les extraits végétaux.

Lorsque l'objectif de l'enrobage est par exemple de protéger le principe actif de l'environnement pendant une période de stockage prolongé, on peut utiliser des polymères de type cellulosique, matières grasses, dérivés d'amidon, ou gomme.

Le matériau d'enrobage protecteur peut également assurer le masquage du goût ou des effets irritants des principes actifs contenus dans le ou les extraits végétaux. On a par exemple obtenu de bons résultats avec un matériau d'enrobage protecteur à base d'hydroxypropylmethylcellulose (HPMC) aqueuse.

Lorsque l'objectif de l'enrobage est d'accélérer/retarder ou cibler la libération des principes actif dans le tractus digestif, le choix du polymère sera fonction de l'espèce animale considérée, de l'objectif recherché (gastrorésistance, libération colique, libération ruminale ou post-ruminale). On peut utiliser par exemple, pour une application chez le monogastrique, des polymères pH sensibles (ou pH dépendants). Une large variété de tels polymères pH sensibles est disponible. Ils se différencient entre eux par le pH à partir duquel ils se dissolvent. Des exemples de matériaux utilisables sont disponibles sous les marques :

- EUDRAGIT®
- AQUATERIC®
- AQOAT®.

Des mélanges de polymères compatibles peuvent être envisagés pour obtenir des dissolutions à pH intermédiaires.

Les seuils des pH de dissolution sont en pratique affectés par le choix de plastifiants, de pigments colorés, incorporés aux polymères, par l'épaisseur de l'enrobage, par la formulation des noyaux et les forces de désintégration.

Toutefois, pour des raisons de coût ou de réglementation, on peut privilégier d'autres excipients (cellulose, cires, graisses ...), qui peuvent retarder la libération du principe actif dans l'environnement digestif, par exemple chez le monogastrique de type chien ou porc.

Le pourcentage d'enrobage à utiliser dépend de la nature de l'excipient, de l'objectif recherché (gastrorésistance, libération colique ...) ainsi que de la taille des particules à enrober.

En pratique, la quantité d'enrobage représente un pourcentage de 10 à 30 % en poids pour des particules de silice sphéroïdale ayant une taille comprise entre 90 μm et 500 μm. On obtient alors une action efficace.

Un plastifiant est souvent exigé dans la composition d'enrobage pour assurer une bonne plasticité du film. Le type de plastifiant utilisé dépendra de la sélection de l'excipient d'enrobage. On peut utiliser un excipient selon une proportion de 5 à 40 %, en fonction des recommandations des fabricants d'excipients.

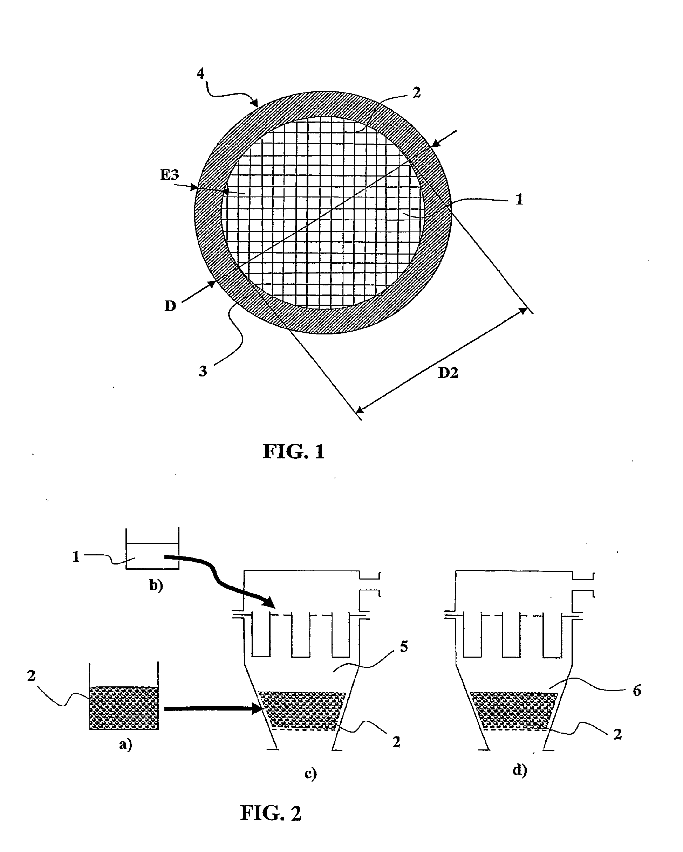

Le produit pulvérulent selon l'invention est illustré sur la figure 1, représentant en coupe diamétrale un grain de produit pulvérulent selon l'invention. On distingue, sur cette coupe, des extraits végétaux sous forme d'un liquide hydrophobe adsorbé et/ou absorbé dans une granule sphéroïdale de silice précipitée 2 dont le diamètre $D_2$ moyen est compris entre 90 µm et 500 µm environ, et on distingue une couche de matériau d'enrobage protecteur 3 ayant une épaisseur $E_3$ et dont la surface extérieure 4 définit le diamètre global D du grain de poudre.

Les extraits végétaux 1 sous forme liquide sont présents dans la silice précipitée 2 selon une proportion supérieure à 20 % en poids.

L'épaisseur $E_3$ du matériau d'enrobage est choisie de façon que le matériau d'enrobage protecteur 3 soit présent en proportion d'environ 10 à 30 % en poids du produit pulvérulent.

On se réfère maintenant à la figure 2, qui illustre les principales étapes du procédé de fabrication d'un produit pulvérulent selon la figure 1.

Selon l'étape a), on prévoit des granules sphéroïdales de silice précipitée 2 de taille moyenne comprise entre 90 µm et 500 µm environ.

Cette silice peut être préparée par un procédé du type comprenant la réaction d'un silicate avec un agent acidifiant pour obtenir une suspension de silice précipitée, puis la séparation et le séchage de cette suspension à l'aide d'un atomiseur à buses. On pourra par exemple utiliser un procédé tel que décrit dans le document WO 99/07237. D'autres procédés pourront être envisagés.

Lors de l'étape b), on prévoit un ou plusieurs extraits végétaux sous forme d'un liquide 1 généralement hydrophobe.

Au cours de l'étape c), on réalise l'adsorption et/ou l'absorption d'une quantité appropriée du liquide hydrophobe 1 dans les granules sphéroïdales de silice précipitée 2.

L'adsorption et/ou l'absorption du liquide 1 sur le support à base de ladite silice précipitée 2 peut s'effectuer par pulvérisation, directement dans un lit d'air fluidisé 5.

En alternative, de manière plus classique, on peut réaliser l'adsorption et/ou l'absorption dans un mélangeur.

La quantité du liquide 1 adsorbée et/ou absorbée dépend en général de l'application recherchée. Toutefois, notamment dans le cas d'une huile essentielle, la teneur en liquide devra être d'au moins 20 % en poids, plus avantageusement supérieure à 30 %, afin d'obtenir des particules de densité suffisante pour permettre l'enrobage ultérieur au cours de l'étape d).

Au cours de l'étape d), on applique, sur les granules sphéroïdales de silice précipitée 2 contenant le liquide hydrophobe 1, au moins une couche de matériau d'enrobage protecteur 3. L'enrobage peut être avantageusement réalisé selon l'invention par pulvérisation du matériau d'enrobage protecteur sur les granules sphéroïdales 2 dans un lit d'air fluidisé 6.

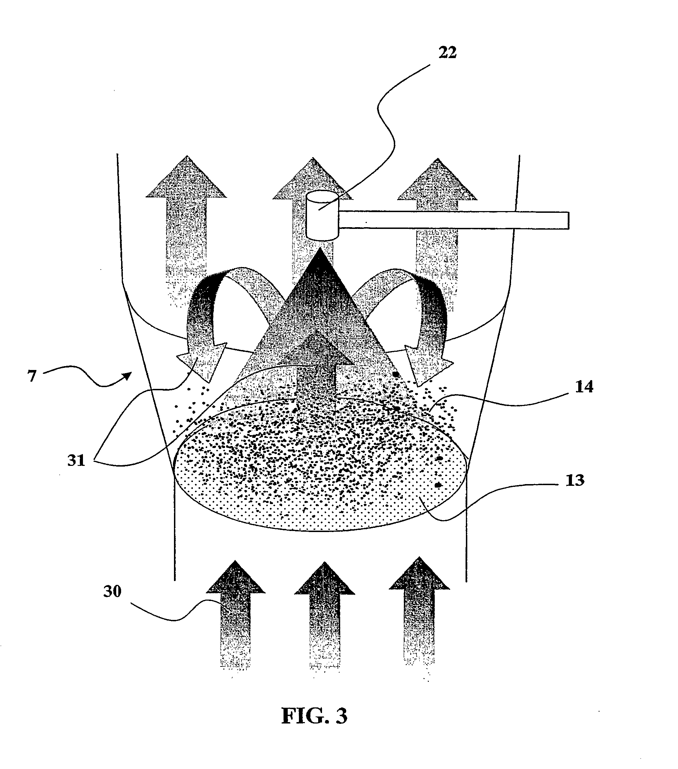

On se réfère maintenant aux figures 3, 4, 5 et 6, pour l'explication de la technique de lit d'air fluidisé.

Le principe du lit d'air fluidisé est basé sur la création d'un flux d'air ascendant de suspension par un phénomène d'aspiration. Ce flux d'air ascendant, dont la pression et la température sont fixées selon des paramètres prédéfinis, est canalisé afin de traverser de bas en haut un lit de matière pulvérulente et d'entraîner sa mise en suspension.

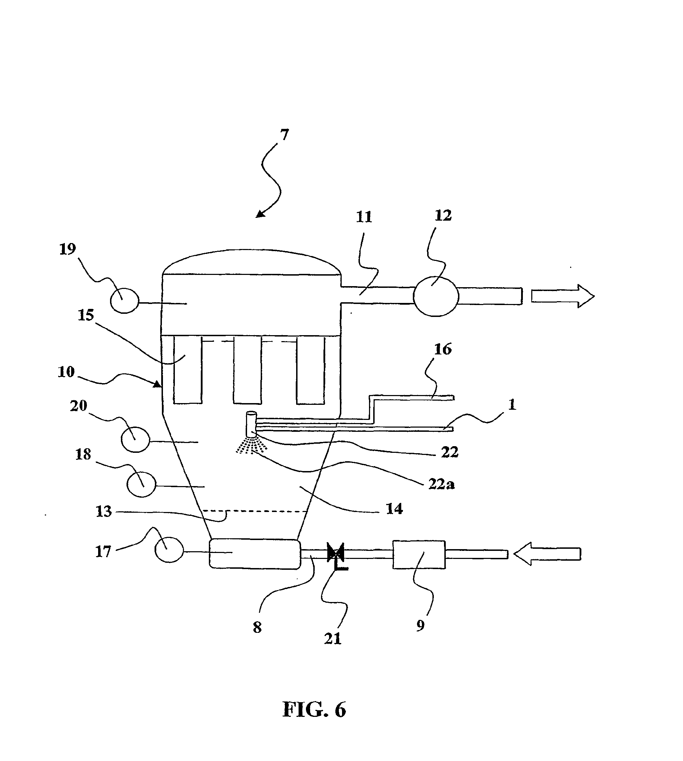

On distingue, sur le système 7 à lit d'air fluidisé selon la figure 6 : une entrée d'air de suspension 8 avec système de filtration et de chauffage 9, laissant entrer de l'air de suspension chaud à la base d'une enceinte 10 ayant une sortie d'air supérieure 11 raccordée à l'atmosphère par un dispositif d'aspiration 12. Une grille de distribution 13, en zone inférieure de l'enceinte 10, limite la base d'une zone de produits pulvérulents fluidisés 14, ou zone de pulvérisation. Au-dessus de la zone de pulvérisation 14 se trouve une zone de filtration 15, en amont de la sortie 11 dans le sens d'écoulement de l'air de suspension.

Des moyens de pulvérisation de liquide sont prévus dans le dispositif pour pulvériser le liquide dans la zone de pulvérisation 14. Dans la réalisation illustrée sur la figure 6, il s'agit d'une pulvérisation depuis le dessus (top spray). La pulvérisation s'effectue par injection de liquide 1 et d'air de pulvérisation 16.

On contrôle la température d'entrée d'air de suspension par des moyens de contrôle de température inférieurs 17, la température dans la zone de pulvérisation 14 par des moyens de contrôle de température intermédiaires 18, la température en zone supérieure par des moyens de contrôle de température supérieurs 19, et l'humidité dans la zone de pulvérisation 14 par des capteurs d'humidité 20. La quantité d'air de suspension admise est contrôlée par une vanne d'entrée d'air 21.

La buse de pulvérisation 22 est placée au-dessus des particules en suspension dans le lit d'air de la zone de pulvérisation 14, et la pulvérisation 22a de l'agent mouillant s'effectue de haut en bas.

L'appareil se compose ainsi de deux sections en contact avec le produit :

- une enceinte 10 ayant avantageusement une forme de cuve conique, équipée d'un fond perforé ou grille 13 pour la retenue du produit à traiter, et équipée de la buse de pulvérisation 22 orientée vers le bas, positionnable à deux niveaux de la cuve selon la quantité de produit traité ;

- une extension supérieure qui forme le logement d'un filtre à manche, constituant la zone de filtration 15 qui permet de retenir le produit en traitement pour éviter son échappement par la sortie d'air supérieure 11.

Périodiquement, pendant le procédé, la fluidisation s'arrête et le filtre est décolmaté par secouage mécanique qui permet aux particules fines de retourner dans le lit d'air pour enrobage.

La figure 3 illustre à nouveau, en perspective partielle, le système 7 de la figure 6, en représentant le flux d'air de suspension 30 et le flux des particules 31 dans la zone de pulvérisation 14.

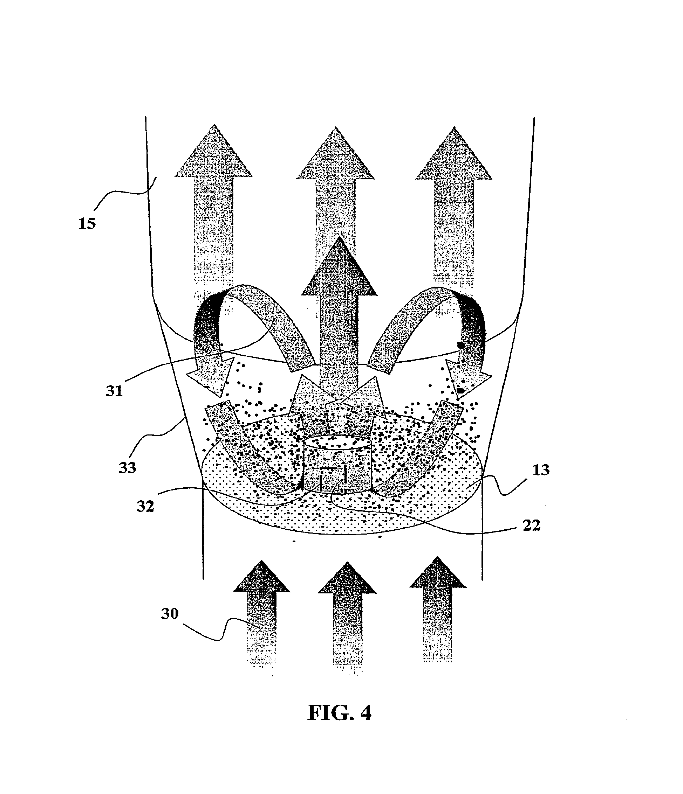

La figure 4 illustre un système d'enrobage à pulvérisation par le bas. La buse de pulvérisation 22 est placée en fond de cuve, au centre de la plaque perforée ou grille 13, et surmontée d'un cylindre 32. La pulvérisation du liquide de mouillage ou de l'agent d'enrobage s'effectue de bas en haut. L'appareil se compose d'une cuve amovible 33 de type WÜRSTER de forme conique s'adaptant sur le logement du filtre à manche 15 de l'appareil. La cuve 33 comporte une plaque de fond de cuve perforée 13, un cylindre 32 (facultatif) situé dans la partie inférieure de la cuve au-dessus de la plaque perforée et dont la hauteur est réglable, et une buse 22 située au centre de la plaque perforée 13, sous le cylindre 32, et orientée pour une pulvérisation de bas en haut. Le volume d'air plus important qui traverse le centre de la plaque perforée 13 et le cylindre 32 interne créent de ce fait un flot ascendant 31 de matière qui redescend ensuite vers l'extérieur en donnant aux particules en suspension un mouvement dit "en fontaine". La matière en suspension circule rapidement de cette façon et, à chaque passage du produit près de la buse de pulvérisation, reçoit une couche d'enrobage supplémentaire. A l'arrêt de la pulvérisation du liquide d'enrobage succède le séchage qui se poursuit dans la même cuve avec simplement une augmentation de la température et du débit de l'air entrant 30. On obtient ainsi une surface très régulière de l'enrobage.

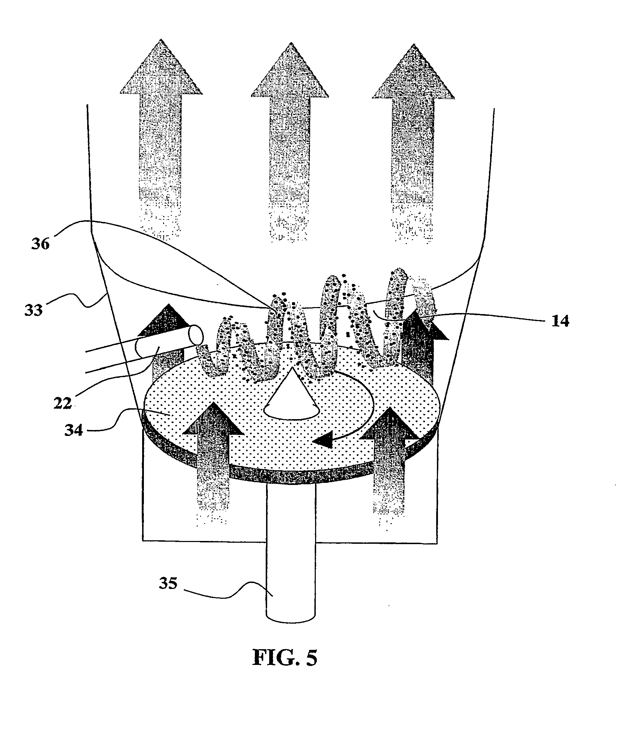

Sur la figure 5, on a illustré un système d'enrobage à pulvérisation tangentielle. Pour cette application particulière, l'appareil est constitué d'une cuve 33 à produits de forme conique, munie à sa base d'un disque métallique 34 monté rotatif autour d'un axe vertical 35 dont la vitesse est modulable. La position verticale du disque peut être modifiée vers le haut ou vers le bas, créant une ouverture plus ou moins grande entre le disque 34 et la cuve à produits 33. L'air pré-conditionné est aspiré à travers cette ouverture plus ou moins grande entre le disque 34 et la cuve 33. Cet air produit la fluidisation des particules à enrober au niveau de la circonférence de la cuve à produits 33. Simultanément, le disque 34 tourne à une certaine vitesse et la force centrifuge générée provoque le déplacement des particules vers les parois de la cuve à produits 33, où celles-ci sont soulevées par le courant d'air fluidisant jusqu'à la chambre d'expansion 14. Les particules sont alors ralenties et redescendent par gravité vers le centre du disque et répètent cycliquement le mouvement. La combinaison de la force centrifuge, de la force ascendante provenant de l'air fluidisant, et de la force gravitationnelle, produit un mouvement hélicoïdal spiralé 36. Le cycle est très rapide et l'effet de mélange est très important. La buse de pulvérisation 22 est immergée au sein du lit de produit fluidisé, et le liquide d'enrobage est appliqué de manière tangentielle vis-à-vis du flux de particules.

Il est important, selon l'invention, de réaliser un enrobage efficace, assurant une protection efficace des extraits végétaux vis-à-vis du milieu ambiant. Pour cela, la couche d'enrobage 3 doit être continue et d'épaisseur suffisante sur toute la surface externe du grain de poudre tel qu'illustré sur la figure 1. L'obtention d'une telle protection est rendue possible par la forme sphéroïdale des granules de silice 2, car le matériau d'enrobage protecteur se répartit alors équitablement sur toute la surface de la granule de silice 2, assurant une épaisseur E3 relativement constante. Simultanément, la quantité de liquide hydrophobe 1 contenue dans la silice assure une densité suffisamment grande, évitant le déplacement excessif des granules dans le lit d'air fluidisé lors de l'enrobage, de sorte que l'enrobage s'effectue de façon satisfaisante.

Egalement, l'enrobage est satisfaisant par le fait de l'absence de particules trop fines dans le lit d'air fluidisé.

On a pu évaluer l'efficacité de l'enrobage par la recherche de la limitation d'un effet irritant de certaines molécules. Des molécules microencapsulées selon l'invention on été appliquées sur la peau d'animaux, sur une zone saine et sur une zone scarifiée. Après des intervalles de temps définis, on a relevé le degré d'irritation. L'essai a été conduit sur une période suffisante de plusieurs jours, de manière à apprécier la réversibilité des effets observés.

C'est ainsi que l'on a pu constater une réduction très sensible des réactions d'irritation par l'utilisation du produit pulvérulent selon l'invention.

On a ensuite pu contrôler la vitesse de dissolution du principe actif à partir de la forme enrobée des produits pulvérulents à base d'extraits végétaux selon l'invention. On a ainsi constaté une vitesse satisfaisante de dissolution.

On donne ci-après un premier exemple de fabrication d'un produit selon l'invention, consistant dans l'adsorption et/ou l'absorption d'eugenol et de cinnamaldéhyde sur un support formé par la silice précipitée. Dans cet exemple, l'eugenol et le cinnamaldéhyde sont des produits de synthèse, chimiquement identiques aux principes actifs contenus dans les extraits d'huiles essentielles de clou de girofle et de cannelle.

La mise sur le support s'est effectuée dans un mélangeur de laboratoire de marque VRIECO-NAUTA de type 020-FFC-50, tournant à 7 tours/minute, avec un axe intérieur tournant à 210 tours/minute, muni d'une buse au travers de laquelle est pulvérisé le liquide en mélange. On a chargé 5 kilogrammes de silice précipitée dans le mélangeur, puis on a pulvérisé 5 kilogrammes de la solution d'eugenol à 62 % et de cinnamaldéhyde à 38 %, à température ambiante et à débit de 75 millilitres/minutes, sur la silice. On a mélangé pendant toute l'opération, puis on a homogénéisé pendant 15 minutes supplémentaires. La composition conditionnée ainsi obtenue contenait 50 % en poids de silice précipitée et 50 % du mélange eugenol et cinnamaldéhyde. On a pu constater que cette composition présentait les propriétés nécessaires pour permettre une opération d'enrobage en lit d'air fluidisé.

L'enrobage de cette composition a été réalisé en lit d'air fluidisé (Aeromatic Fieder MP1). La pulvérisation a été réalisée par un système de pulvérisation par le haut. Le produit d'enrobage était à base d'hydroxypropylmethylcellulose (HPMC) aqueuse, disponible sur le marché sous la marque Pharmacoat 603, et appliquée sur la silice selon le procédé ci-après. On a préparé la solution d'enrobage, sous forme de mélange d'HPMC (18 %) et de polyethylglycol 6 000 (2 %), réalisée dans l'eau sous agitation vigoureuse jusqu'à l'obtention d'une dispersion homogène, puis en laissant au repos pendant 24 heures. Une masse de 1 000 grammes de la composition conditionnés à base d'un support silice a été mise dans la cuve du lit d'air fluidisé, puis une masse de 556 grammes de la solution d'enrobage a été pulvérisée afin d'obtenir un pourcentage d'enrobage de 10 %. Le débit d'air de suspension dans l'appareil d'enrobage était de 80 à 90 $m^3$/heure. La température de la solution d'enrobage était de 25°C. La température du produit était de 25 à 35°C. La pression d'air de pulvérisation était de 2,5 bars. Le débit de pulvérisation était de 15 g/minute. Le diamètre de la buse était de 1 mm. Le temps de séchage après pulvérisation de la solution d'enrobage a été de 5 à 10 minutes.

On donne ci-dessous un second exemple de fabrication d'un produit selon l'invention. La première étape d'adsorption et/ou absorption a été identique à celle de l'exemple précédent. La seconde étape d'enrobage a été réalisée en lit d'air fluidisé à base d'un support silice avec l'appareil Aeromatic Fieder MP1. Le produit d'enrobage a été une émulsion d'ethylcellulose (EC) de marque Aquacoat ECD comme excipient d'enrobage. Une solution comprenant 614,9 grammes d'Aquacoat ECD et 45,76 grammes de Dibutylsebacat complétés par 877,3 grammes d'eau a été réalisée puis laissée au repos pendant 24 heures.

Une masse de 800 grammes de la composition conditionnée à base d'un support silice a été mise dans une cuve à lit d'air fluidisé. Le débit d'air de l'appareil a été fixé à 80-90 $m^3$/h, la température de la solution d'enrobage était de 25°C, la température du produit était de 25 à 35°C, la pression d'air de pulvérisation a été de 2,5 bars, pour un débit de pulvérisation de 15 grammes/minutes, et un diamètre de buse de 1 mm. Une masse de 200 grammes de la solution d'enrobage a été pulvérisée afin d'obtenir un pourcentage d'enrobage de 10 %. Après pulvérisation de la solution d'enrobage, on a respecté un temps de séchage de 5 à 10 minutes.

On donne ci-dessous un troisième exemple de fabrication d'un produit selon l'invention. La première étape d'adsorption et/ou absorption a été identique à celle des exemples précédents. La seconde étape d'enrobage a été réalisée en lit d'air fluidisé à base d'un support silice avec l'appareil Aeromatic Fielder MP1. Le produit d'enrobage a été une huile de colza hydrogénée comme excipient d'enrobage.

Une masse de 900 grammes de la composition conditionnée à base d'un support silice a été mise dans une cuve à lit d'air fluidisé. Le débit d'air de l'appareil a été fixé à 90 $m^3$/h, la température de la solution d'enrobage était de 80°C, la température du produit était de 37-42°C, la pression d'air de pulvérisation a été de 2.5 bars, pour un débit de pulvérisation de 18 grammes/minutes, et un diamètre de buse de 1 mm. Une masse de 100 grammes de la solution d'enrobage a été pulvérisée afin d'obtenir un pourcentage d'enrobage de 10 %. Après pulvérisation de la solution d'enrobage, on a respecté un temps de cristallisation de l'excipient d'enrobage de 5 à 10 minutes.

La présente invention n'est pas limitée aux modes de réalisation qui ont été explicitement décrits, mais elle en inclut les diverses variantes et généralisations contenues dans le domaine des revendications ci-après.

**Revendications**

**1.** - Produit pulvérulent à base d'un ou plusieurs extraits végétaux pour la nutrition animale ou la médecine vétérinaire, dans lequel :

- le ou les extraits végétaux sont adsorbés et/ou absorbés dans de la silice précipitée,
- la silice précipitée est sous forme de granules sphéroïdales (2) non agglomérées,
- les granules sphéroïdales (2) de silice précipitée ont une taille comprise entre 90 $\mu$m et 500 $\mu$m environ, de préférence comprise entre 200 $\mu$m et 500 $\mu$m environ, sensiblement dépourvues de fines particules,

**caractérisé en ce que** :

- le ou les extraits végétaux sont sous forme de liquide hydrophobe (1),
- les granules sphéroïdales de silice précipitée (2) contenant le ou les extraits végétaux adsorbés et/ou absorbés sont enrobées d'une couche de matériau d'enrobage protecteur (3),

- le ou les extraits végétaux (1) sous forme de liquide hydrophobe sont présents dans la silice précipitée (2) selon une proportion supérieure à 20 % en poids.

2. - Produit selon la revendication 1, **caractérisé en ce que** le ou les extraits végétaux (1) sous forme liquide sont présents dans la silice précipitée (2) selon une proportion supérieure à 30 % en poids.

3. - Produit selon l'une des revendications 1 ou 2, **caractérisé en ce que** les granules sphéroïdales (2) de silice précipitée ont une densité de remplissage à l'état tassé DRT supérieure à 0,29, une prise d'huile DOP supérieure à 100 milfilitres/ 100 grammes, une surface BET comprise entre 140 et 240 m$^2$/gramme environ, une surface spécifique CTAB comprise entre 140 et 230 m$^2$/gramme environ, un taux d'humidité inférieur à 5 % en poids, un taux de refus au tamis d'au moins 92 % en poids pour des ouvertures de maille de 75 $\mu$m.

4. - Produit selon l'une quelconque des revendications 1 à 3, **caractérisé en ce que** le matériau d'enrobage protecteur (3) est présent en proportion d'environ 10 à 30 % en poids du produit pulvérulent.

5. - Produit selon l'une quelconque des revendications 1 à 4, **caractérisé en ce que** le matériau d'enrobage protecteur (3) assure le masquage du goût ou des effets irritants des principes actifs contenus dans le ou les extraits végétaux.

6. - Produit selon l'une quelconque des revendications 1 à 5, **caractérisé en ce que** le matériau d'enrobage protecteur (3) assure la stabilité des principes actifs contenus dans le ou les extraits végétaux pendant un temps de stockage et/ou lors de procédés industriels d'utilisation du produit.

7. - Produit selon l'une quelconque des revendications 1 à 6, **caractérisé en ce que** le matériau d'enrobage protecteur (3) assure une libération accélérée, retardée ou ciblée, dans le tractus digestif, des principes actifs contenus dans les extraits végétaux.

8. - Produit selon l'une quelconque des revendications 1 à 7, **caractérisé en ce que** le matériau d'enrobage protecteur (3) est choisi parmi les agents d'enrobage utilisés dans les domaines pharmaceutique ou agro-alimentaire.

9. - Produit selon l'une quelconque des revendications 1 à 8, **caractérisé en ce que** le matériau d'enrobage protecteur (3) est à base de gomme, ou de polysaccharides (amidon, cellulose), ou de protéine, ou de copolymères d'acide méthacrylique, ou de matière grasse, ou de leurs mélanges.

10. - Produit selon l'une quelconque des revendications 1 à 9, **caractérisé en ce que** la couche de matériau d'enrobage protecteur (3) présente les propriétés physico-chimiques d'une couche réalisée par lit d'air fluidisé.

11. - Produit selon l'une quelconque des revendications 1 à 10, **caractérisé en ce que** le ou les extraits végétaux sont initialement sous forme de liquide hydrophobe tel que huile essentielle, oléorésine, gomme, résine, arôme.

12. - Procédé de fabrication d'un produit pulvérulent selon l'une quelconque des revendications 1 à 11, **caractérisé en ce qu'**il comprend les étapes :

    a) prévoir des granules sphéroïdales de silice précipitée (2) de taille moyenne comprise entre 90 $\mu$m et 500 $\mu$m environ, de préférence comprise entre 200 $\mu$m et 500 $\mu$m environ, sensiblement dépourvues de fines particules,
    b) prévoir un ou plusieurs extraits végétaux sous forme de liquide hydrophobe (1),
    c) réaliser l'adsorption et/ou l'absorption d'une quantité d'au moins 20 % en poids du liquide hydrophobe (1) dans les granules sphéroïdale de silice précipitée (2), en évitant leur agglomération,
    d) appliquer, sur les granules sphéroïdales non agglomérées de silice précipitée (2) contenant le liquide hydrophobe (1), au moins une couche de matériau d'enrobage protecteur (3) par pulvérisation du matériau d'enrobage protecteur (3) liquide sur les granules sphéroïdales (2) dans un lit d'air fluidisé (6).

13. - Procédé selon la revendication 12, **caractérisé en ce que**, lors de l'étape d), le matériau d'enrobage protecteur (3) liquide est une solution aqueuse, ou une émulsion aqueuse, ou une graisse (lipide).

14. - Procédé selon l'une des revendications 12 ou 13, **caractérisé en ce que**, dans le matériau d'enrobage protecteur en émulsion aqueuse ou en solution aqueuse, la concentration en excipient d'enrobage est de 8 % à 30 % en poids; et de préférence de 10 % à 25 % en poids.

**15.** - Procédé selon l'une quelconque des revendications 12 à 14, **caractérisé en ce que** le matériau d'enrobage protecteur (3) contient un plastifiant en proportion de 5 à 40 % en poids de matériau.

**16.** - Procédé selon l'une quelconque des revendications 12 à 15, **caractérisé en ce que** l'étape c) d'adsorption et/ou d'absorption est effectuée par pulvérisation dans un lit d'air fluidisé (5) ou dans un élangeur.

**17.** - Procédé selon l'une quelconque des revendications 12 à 16, **caractérisé en ce que**, lors de l'étape c), le liquide hydrophobe (1) est adsorbé et/ou absorbé dans la silice précipitée (2) selon une quantité d'au moins 30 % en poids.

**18.** - Application d'un produit selon l'une quelconque des revendications 1 à 11 à la nutrition animale, dans laquelle on introduit en faible quantité le produit pulvérulent dans un substrat alimentaire.

**Patentansprüche**

**1.** - Pulverförmiges Produkt auf Basis von einem oder mehreren Pflanzenextrakt(en) für Tiernahrung oder Tiermedizin, bei dem:

- das oder die Pflanzenextrakt(e) adsorbiert sind und/oder in ausgefällter Kieselsäure absorbiert sind,
- die ausgefällte Kieselsäure in der Form von nicht agglomerierten sphäroidischem Granulat (2) ist,
- das sphäroidische Granulat (2) der ausgefällten Kieselsäure eine Größe zwischen ungefähr 90 $\mu$m und 500 $\mu$m, vorzugsweise zwischen ungefähr 200 $\mu$m und 500 $\mu$m hat, das im wesentlichen frei von feinen Partikeln ist,

**dadurch gekennzeichnet, dass**:

- das oder die Pflanzenextrakt(e) die Form einer hydrophoben Flüssigkeit (1) hat/haben,
- das sphäroidale Granulat der ausgefällten Kieselsäure (2) das oder die adsorbierten und/oder absorbierten Pflanzenextrakt(e) enthält, die mit einer Schicht aus einem Material einer Schutzumhüllung (3) beschichtet ist/sind,
- das oder die Pflanzenextrakt(e) (1) in Form einer hydrophoben Flüssigkeit in einem Verhältnis von mehr als 20 Gewichts% in der ausgefällten Kieselsäure (2) vorhanden ist/sind.

**2.** - Produkt nach Anspruch 1, **dadurch gekennzeichnet, dass** das oder die Pflanzenextrakt(e) (1) in flüssiger Form in der ausgefällten Kieselsäure (2) in einem Verhältnis von mehr als 30 Gewichts% vorhanden ist/sind.

**3.** - Produkt nach einem der Ansprüche 1 oder 2, **dadurch gekennzeichnet, dass** das sphäroidale Granulat (2) von ausgefällter Kieselsäure eine Füllungsdichte in zusammengepressten Zustand DRT von mehr als 0,29, eine Prise von Öl DOP von mehr als 100 ml/100 gramm, eine Oberfläche BET zwischen ungefähr 140 und 240 m$^2$/gramm, eine spezifische Oberfläche CTAB zwischen ungefähr 140 und 230 m$^2$/gramm, eine Feuchtigkeitsrate von weniger als 5 Gewichts%, und eine Rückstandsrate in einem Sieb von mindestens 92 Gewichts% bei Öffnungen der Maschen von 75 $\mu$m.

**4.** - Produkt nach irgendeinem der Ansprüche 1 bis 3, **dadurch gekennzeichnet, dass** das Material der Schutzumhüllung (3) in einem Verhältnis von ungefähr 10 bis 30 Gewichts% des pulverförmigen Produktes vorhanden ist.

**5.** - Produkt nach irgendeinem der Ansprüche 1 bis 4, **dadurch gekennzeichnet, dass** das Material der Schutzumhüllung (3) eine Verdeckung des Geschmacks oder von irritierenden Effekten von aktiven Ingredienzien, die in den Pflanzenextrakten vorhanden sind, sicherstellt.

**6.** - Produkt nach irgendeinem der Ansprüche 1 bis 5, **dadurch gekennzeichnet, dass** das Material der Schutzumhüllung (3) die Stabilität der aktiven Ingredienzien sicherstellt, die in dem oder den Pflanzenextrakt(en) enthalten sind, während einer Lagerzeit und/oder während der industriellen Prozesse zur Verwendung des Produktes.

**7.** - Produkt nach irgendeinem der Ansprüche 1 bis 6, **dadurch gekennzeichnet, dass** das Material der Schutzumhüllung (3) eine beschleunigte, verzögerte oder gezielte Freisetzung im Verdauungstrakt der aktiven Ingredienzien sicherstellt, die in den Pflanzenextrakten vorhanden sind.

**8.** - Produkt nach irgendeinem der Ansprüche 1 bis 7, **dadurch gekennzeichnet, dass** das Material der Schutzumhüllung (3) aus Beschichtungsmitteln ausgewählt ist, die in pharmazeutischen oder Agrarnahrungsmitteln verwendet werden.

**9.** - Produkt nach irgendeinem der Ansprüche 1 bis 8, **dadurch gekennzeichnet, dass** das Material der Schutzumhüllung (3) auf Basis von Gummi, oder Polysacchariden (Stärke, Zellulose) oder Proteinen oder Copolymeren von Methacrylsäure oder Fettmasse oder deren Mischungen ist.

**10.** - Produkt nach irgendeinem der Ansprüche 1 bis 9, **dadurch gekennzeichnet, dass** die Schicht aus Material der Schutzumhüllung (3) physiko-chemische Eigenschaften einer Schicht aufweist, die durch ein fluidisiertes Luftbett erzeugt wird.

**11.** - Produkt nach irgendeinem der Ansprüche 1 bis 10, **dadurch gekennzeichnet, dass** die vegetarischen Extrakte anfänglich in Form einer hydrophoben Flüssigkeit vorliegen, wie z.B. essentiellen Ölen, Ölharz, Gummiharz, Aroma.

**12.** - Verfahren zur Herstellung eines pulverförmigen Produktes nach einem oder mehreren der Ansprüche 1 bis 11, **dadurch gekennzeichnet, dass** es folgende Schritte aufweist:

a) Bereitstellen von sphäroidalem Granulat von ausgefällter Kieselsäure (2) mit einer mittleren Größe von zwischen ungefähr 90 $\mu$m und 500 $\mu$m, vorzugsweise zwischen ungefähr 200 $\mu$m und 500 $\mu$m, das im wesentlichen aus feinen Partikeln besteht,
b) Bereitstellen von einem oder mehreren Pflanzenextrakt(en) in Form einer hydrophoben Flüssigkeit (1),
c) Bewirken einer Adsorption und/oder einer Absorption einer Menge von mindestens 20 Gewichts% der hydrophoben Flüssigkeit (1) in dem spähroidalen Granulat der ausgefällten Kieselsäure (2) und Vermeiden deren Agglomeration,
d) Auftragen auf das nicht agglomerierte sphäroidale Granulat (2) von ausgefällter Kieselsäure, die die hydrophobe Flüssigkeit (1) enthält, von mindestens einer Schicht eines Materiales einer Schutzumhüllung (3) durch Zerstäuben eines flüssigen Materiales der Schutzumhüllung (3) auf das sphäroidale Granulat (2) in einem fluidisierten Luftbett (6).

**13.** - Verfahren nach Anspruch 12, **dadurch gekennzeichnet, dass** während des Schrittes d) das flüssige Material der Schutzumhüllung (3) eine wässrige Lösung oder eine wässrige Emulsion oder ein Fett (Lipid) ist.

**14.** - Verfahren nach einem der Ansprüche 12 oder 13, **dadurch gekennzeichnet, dass** in dem Material der Schutzumhüllung in einer wässrigen Emulsion oder in einer wässrigen Lösung die Konzentration des Beschichtungshilfstoffes bei 8 Gewichts% bis 30 Gewichts% liegt und vorzugsweise von 10 Gewichts% bis 25 Gewichts%.

**15.** - Verfahren nach irgendeinem der Ansprüche 12 bis 14, **dadurch gekennzeichnet, dass** das Material der Schutzumhüllung (3) ein Weichmacher im Verhältnis von 5 bis 40 Gewichts% des Materiales ist.

**16.** - Verfahren nach irgendeinem der Ansprüche 12 bis 15, **dadurch gekennzeichnet, dass** der Schritt c) der Adsorption und/oder der Absorption durch Versprühen in einem fluidisiertem Luftbett (5) oder in einem Mischer ausgeführt wird.

**17.** - Verfahren nach einem der Ansprüche 12 bis 16, **dadurch gekennzeichnet, dass** während des Schrittes c) die hydrophobe Flüssigkeit (1) in der ausgefällten Kieselsäure (2) in einer Menge von 30 Gewichts% adsorbiert und/oder absorbiert ist.

**18.** - Verwendung eines Produktes nach irgendeinem der Ansprüche 1 bis 11 als Tiernahrung, der man eine geringe Menge des pulverförmigen Produktes in ein Nahrungssubstrat zugibt.

## Claims

**1.** Pulverulent product based on one or more plant extracts for animal feed or veterinary medicine, in which:

- the plant extract(s) is (are) adsorbed and/or absorbed in precipitated silica,
- the precipitated silica is in the form of nonagglomerated spheroidal granules (2),
- the spheroidal granules (2) of precipitated silica have a size of between approximately 90 $\mu$m and 500 $\mu$m, preferably between approximately 200 $\mu$m and 500 $\mu$m, substantially devoid of fine particles,

**characterized in that**:

- the plant extract(s) is (are) in the form of a hydrophobic liquid (1),
- the spheroidal granules of precipitated silica (2) containing the adsorbed and/or absorbed plant extract(s) are coated with a layer of protective coating material (3),
- the plant extract(s) (1) in hydrophobic liquid form is (are) present in the precipitated silica (2) according to a proportion of greater than 20% by weight.

2. Product according to claim 1, **characterized in that** the plant extract(s) (1) in liquid form is (are) present in the precipitated silica (2) according to a proportion of greater than 30% by weight.

3. Product according to either of claim 1 or claim 2, **characterized in that** the spheroidal granules (2) of precipitated silica have a fill density in the packed state DRT of greater than 0.29, a DOP oil uptake of greater than 100 milliliters/100 grams, a BET surface area of between approximately 140 and 240 $m^2$/gram, a CTAB specific surface area of between approximately 140 and 230 $m^2$/gram, a water content of less than 5% by weight, and a screen oversize rate of at least 92% by weight for mesh apertures of 75 $\mu$m.

4. Product according to any one of claims 1 to 3, **characterized in that** the protective coating material (3) is present in a proportion of approximately 10% to 30% by weight of the pulverulent product.

5. Product according to any one of claims 1 to 4, **characterized in that** the protective coating material (3) provides masking of the taste or of the irritant effects of the active ingredients contained in the plant extract(s).

6. Product according to any one of claims 1 to 5, **characterized in that** the protective coating material (3) ensures the stability of the active ingredients contained in the plant extract(s) over a period of storage and/or during industrial processes for use of the product.

7. Product according to any one of claims 1 to 6, **characterized in that** the protective coating material (3) provides accelerated, delayed or targeted release, in the digestive tract, of the active ingredients contained in the plant extracts.

8. Product according to any one of claims 1 to 7, **characterized in that** the protective coating material (3) is chosen from the coating agents used in pharmaceuticals or agrofoods.

9. Product according to any one of claims 1 to 8, **characterized in that** the protective coating material (3) is based on gum, or on polysaccharides (starch, cellulose), or on protein, or on methacrylic acid copolymers, or on fats, or on mixtures thereof.

10. Product according to any one of claims 1 to 9, **characterized in that** the layer of protective coating material (3) has the physicochemical properties of a layer produced by a fluidized airbed.

11. Product according to any one of claims 1 to 10, **characterized in that** the plant extract(s) is (are) initially in the form of a hydrophobic liquid such as an essential oil, oleoresin, gum, resin or aroma.

12. Process for the manufacture of a pulverulent product according to any one of claims 1 to 11, **characterized in that** it comprises the following steps:

a) providing spheroidal granules of precipitated silica (2) with an average size of between approximately 90 $\mu$m and 500 $\mu$m, preferably between approximately 200 $\mu$m and 500 $\mu$m, substantially devoid of fine particles,
b) providing one or more plant extracts in the form of a hydrophobic liquid (1),
c) carrying out the adsorption and/or the absorption of an amount of at least 20% by weight of the hydrophobic liquid (1) in the spheroidal granules of precipitated silica (2), while at the same time preventing agglomeration of said granules,
d) applying at least one layer of protective coating material (3) to the nonagglomerated spheroidal granules of precipitated silica (2) containing the hydrophobic liquid (1) by spraying the liquid protective coating material (3) onto the spheroidal granules (2) in a fluidized airbed (6).

13. Process according to claim 12, **characterized in that**, during step d), the liquid protective coating material (3) is an aqueous solution, or an aqueous emulsion, or a fat (lipid).

14. Process according to either of claim 12 or claim 13, **characterized in that**, in the protective coating material in an aqueous emulsion or in an aqueous solution, the concentration of coating excipient is from 8% to 30% by weight, and preferably from 10% to 25% by weight.

15. Process according to any one of claims 12 to 14, **characterized in that** the protective coating material (3) contains a plasticizer in a proportion of from 5% to 40% by weight of material.

16. Process according to any one of claims 12 to 15, **characterized in that** the adsorption and/or absorption step c) is carried out by spraying in a fluidized airbed (5) or in a mixer.

17. Process according to any one of claims 12 to 16, **characterized in that**, during step c), the hydrophobic liquid (1) is adsorbed and/or absorbed in the precipitated silica (2) according to an amount of at least 30% by weight.

18. Application of a product according to any one of claims 1 to 11 to animal feed, in which a small amount of the pulverulent product is introduced into a feed substrate.

**FIG. 1**

**FIG. 2**

**FIG. 3**

**FIG. 4**

**FIG. 5**

**FIG. 6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004073689 A1
- US 20030003040 A1
- WO 9907237 A
- EP 0345109 A1
- GB 1125882 A
- EP 1132009 A

**Littérature non-brevet citée dans la description**

- **BRUNAUER - EMMET - TELLER.** *The journal of the American Chemical Society,* Février 1938, vol. 60, 309